(19) Europäisches Patentamt

European Patent Office

Office européen des brevets

(11) Publication number: **0 164 117 B1**

(12) **EUROPEAN PATENT SPECIFICATION**

(45) Date of publication of patent specification: 26.06.91

(51) Int. Cl.⁵: **C12N 15/00, C12N 1/20,** //(C12N1/20,C12R1:125)

(21) Application number: 85106994.8

(22) Date of filing: 05.06.85

(54) **Amylase-negative, asporogenous mutant of Bacillus subtilis useful as a host in a host-vector system.**

(30) Priority: 06.06.84 US 617874

(43) Date of publication of application:
11.12.85 Bulletin 85/50

(45) Publication of the grant of the patent:
26.06.91 Bulletin 91/26

(84) Designated Contracting States:
AT BE CH DE FR IT LI NL SE

(56) References cited:
EP-A- 0 092 233
EP-A- 0 092 234
EP-A- 0 092 235
EP-A- 0 134 048

(73) Proprietor: CPC INTERNATIONAL INC.
International Plaza P.O. Box 8000
Englewood Cliffs New Jersey 07632(US)

(72) Inventor: Davis, Philip E.
8235 "D" Portsmouth
Darien Illinois 60559(US)
Inventor: Mielenz, Jonathan R.
3499 Nancy Place
Shoreview Minnesota 55112(US)

(74) Representative: Lederer, Franz, Dr.
Lederer, Keller & Riederer Patentanwälte
Lucile-Grahn-Strasse 22
W-8000 München 80(DE)

## Description

This invention relates to a new amylase-negative mutant of Bacillus subtilis useful as a host into which vectors containing amylase-coding genes can be introduced using recombinant DNA methodology.

Most genetic material in a bacterium exists as giant DNA molecules which are present as the chromosome of the cell. A certain amount of the genetic material may also be present in the form of smaller, closed circular DNA molecules known as plasmids. The portion of the DNA molecule related to a specific hereditary trait is called a gene.

By techniques referred to as genetic engineering, it is possible to transfer a gene, which codes for the production of a specific protein, from one microorganism to another. The microorganism which receives the new genetic material is referred to as the host. Various workers have used these techniques to provide microorganisms which are superior producers of certain specific proteins such as enzymes.

It has been discovered that plasmids, which contain a series of genes linked together in the form of a circle, can be removed from the cells of one microorganism and inserted into the cells of another microorganism with comparative ease. Plasmids can also be used as vectors to carry new genetic material into a host organism. This is accomplished by first cutting the plasmid with an enzyme, known as a restriction endonuclease, that opens the circle of DNA. A fragment of foreign DNA, containing the desired gene, is inserted into the place where the DNA circle was cut. The circle is reformed by treatment with DNA ligase. The recombined plasmid, a new circular DNA molecule, contains the genes of the original plasmid plus the new gene from the piece of DNA which was inserted. This plasmid can be introduced into a host microorganism. The plasmid containing the new gene is then reproduced in the host microorganism and becomes part of its genetic material.

For a host microorganism to be suitable for use in genetic engineering, it must be capable of incorporating the new DNA. Furthermore, it must yield a viable microorganism which expresses the traits coded in the newly inserted gene. For the microorganism to produce useful quantities of protein, the microorganism must also be one that can be grown on a commercial scale.

Experimenters using the new recombinant DNA technology have been concerned that the microorganisms with new genetic material might produce substances harmful to man, animals or plants. Because of this concern, the National Institute of Health (NIH) issued "Guidelines for Research Involving Recombinant DNA Molecules" in 1978.

These guidelines provided for various levels of physical containment in laboratories where genetic engineering experiments are conducted. They also established levels of biological containment for microorganisms containing recombinant DNA.

Biological containment relates to the use of host cells and vectors which have limited ability to survive if they escape from the laboratories into the natural environment. Cells of microorganisms which do not form spores (i.e., ones that are asporogenous) have such a limited ability to survive.

The present invention describes a new asporogenous mutant of B. subtilis BI-109 useful as host in a host-vector system. This host has the additional advantage of being an amylase-negative mutant. The mutant is particularly suitable for use as a host for recombinant plasmids which contain genes coding for the production of specific amylases such as thermostable alpha-amylases. These amylases are of commercial importance for use in processes to make starch hydrolyzates, glucose, and high fructose syrups.

The mutant of the present invention readily incorporates plasmids containing a thermostable alpha-amylase gene. The resulting microorganism is a superior producer of thermostable alpha-amylase enzyme to host-vector systems prepared from previously described amylase-negative strains of B. subtilis such as ATCC 39,096, disclosed in published European Patent Application No. 092 235, issued on October 26, 1983.

In accordance with this invention, there is provided a culture of asporogenous B. subtilis BI-109 suitable for use as a host component in a host-vector system characterized in that it contains no amylase-coding gene, has a frequency of reversion to spore formers of less than about $10^{-7}$ when grown under conditions of aeration and having the following genetic markers: spoIIA12, amyE, and SacA321. A strain of B. subtilis having these characteristics has been deposited with the American Type Culture Collection as ATCC 39,701.

In addition, there is provided a culture of amylase-negative B. subtilis BI-20 suitable for use as a host component in a host-vector system and useful as an intermediate for preparation of amylase-negative host components of host-vector systems characterized in that it contains the following genetic markers: metB5, amyE, and sacA321. A strain of B. subtilis having these characteristics has been deposited with the American Type Culture Collection as ATCC 39,706.

Also provided in accordance with this invention is a process for preparing a strain of B. subtilis having the genetic markers: metB5, amyE, and sacA321, which comprises transforming competent cells of B. subtilis Strain IA289 with DNA from B. subtilis Strain IA221 and isolating cells that do not produce alpha-amylase enzyme.

Further provided in accordance with this invention is a process for preparing a strain of B. subtilis having the genetic markers: spoIIA12, amyE, and sacA321, which comprises transferring competent cells of B. subtilis Strain BI-20 with DNA from B. subtilis strain IS30 and isolating cells that grow in the absence of added methionine, do not produce alpha-amylase enzyme, and that do not form spores when heat shocked at 90° C for 10 minutes.

Finally, there is provided a process for using the strains of B. subtilis BI-109 and BI-20 as hosts in host-vector systems which comprises transforming a plasmid containing desired genetic material into competent cells of one of the B. subtilis strains and growing the cells under conditions whereby the plasmid is maintained within the cells.

The B. subtilis strains disclosed and claimed were prepared by incorporating the genetic material from other strains of B. subtilis. An amylase-negative strain of B. subtilis IA289 (ATCC 39,711) containing the markers: arol906, metB5, sacA321, and amyE was transformed into a strain no longer requiring aromatic amino acids by incorporation of a portion of the DNA from B. subtilis Strain IA221 (ATCC 39,086).

Strain IA289 was reported by Steinmetz, et al, Mol. Gen. Genet., 148, 281-285 (1976). It is available from the American Type Culture Collection, Rockville, Maryland, as ATCC 39,711.

Strain IA221 was reported by Dubnau and Smith, Proc. Natl. Acad. Sci., U.S.A., 65, 96-103 (1970). It is available from the American Type Culture Collection, Rockville, Maryland, as ATCC 39,086.

The resultant transformant, designated as BI-20, contains the markers: metB5, amyE, and sacA321. It is available from the American Type Culture Collection as ATCC 39,706. This amylase-negative strain is useful as an intermediate for the preparation of amylase-negative host components of host-vector systems. It can also serve as a host itself under conditions where a spore-forming host is acceptable.

A portion of the DNA from an asporogenic strain of B. subtilis was transferred into Strain BI-20 to produce new Strain BI-109. The asporogenic strain employed as the DNA donor was IS30 obtained from the Bacillus Genetic Stock Center, Dept. of Microbiology, Columbus, Ohio. This strain which contains the marker spoIIA12, was described by Ionesco and Schaeffer, Ann. Inst. Pasteur, Paris, 114, 1-9 (1968). It is available from the American Type Culture Collection, Rockville, Maryland, as ATCC 39,712.

The asporogenous, amylase-negative strain of the present invention, BI-109, shows a frequency of reversion to spore formers of less than about $10^{-7}$.

It is able to grow under industrial conditions and does not require expensive growth additives. It has a low survival rate under natural or escape conditions and a very low tendency to transmit plasmids to other organisms by natural genetic transfer. The organism shows a high degree of competence for plasmid uptake when subjected to classical transformation techniques. Excellent transformations were achieved using competent cell transformation procedures. It functions well as a host for various plasmid vectors making it useful as a host component of a B. subtilis host-vector system. Being amylase-negative, it is particularly suitable for use as a host for recombinant plasmids which contain genes coding for the production of amylases.

A detailed genetic map of the B. subtilis chromosome has been published by Henner and Hoch, Microbiological Reviews, 44, 57-82 (1980). Brief description of the genetic markers mentioned in the disclosure of this invention follows:

(1) spoIIA12: This is a deletion mutation causing a block in the earliest stages of sporulation. This mutation destroys the ability of the Bacillus to form spores, the form in which it normally survives in nature when subjected to heat, ultraviolet light, chemicals and desiccation.

(2) arol906: This is a mutation which causes the mutants to require phenylalanine, tyrosine, and tryptophan. When deprived of these amino acids, a strain carrying this mutant ceases to grow.

(3) amyE: This marker is associated with a deficiency in the structural gene for alpha-amylase production. It is characterized by a very low reversion frequency.

(4) lin2: Strains containing this genetic marker will grow when plates containing minimal medium supplemented with appropriate amino acids and containing lincomycin at a concentration of 100 μg/ml. Strains which do not contain the marker will not grow on such plates.

(5) metB5: This mutation causes the mutants to require methionine. When deprived of this amino acid, a strain carrying this mutant ceases to grow.

(6) sacA321: This is a mutation that prevents formation of an enzyme essential to the metabolism of sucrose by the microorganism. This prevents the host from growing on a medium which contains sucrose as the carbon source.

The following example illustrates certain embodiments of the present invention. Unless otherwise stated, all proportions and percentages are provided on the basis of weight. All strains bearing ATCC numbers are available from the American Type Culture Collection, Rockville, Maryland. These strains have been deposited under the provisions of the Budapest Treaty for deposits of microorganisms for patent purposes. All reagents bearing the Difco name are available from the Difco Laboratories, Detroit, Michigan.

EXAMPLE

Transformation of B. subtilis Strain IA289 (ATCC 39,711) into a strain no longer requiring supplemental aromatic amino acids for growth was accomplished by incorporating DNA obtained from B. subtilis Strain IA221 (ATCC 39,086) as follows.

Cells of B. subtilis Strain IA289 (ATCC 39,711) were grown overnight on plates containing Tryptose Blood Agar Base[R] (Difco). Cells from these plates were inoculated into Penassay Broth[R] (Difco) and grown overnight at 37°C with shaking at 200 rpm. The cells were separated by centrifugation and suspended in 5 volumes of a growth medium containing 0.5% glucose, 0.6% $KH_2PO_4$, 1.4% $K_2HPO_4$, 0.1% sodium citrate, 0.2% $(NH_4)_2SO_4$ and 0.07% $MgSO_4$ supplemented with leucine, isoleucine and methionine at a level of 50 μg/ml and with histidine, tryptophan, arginine, valine, lysine, threonine, glycine and aspartic acid at a level of 25 μg/ml. The increase in optical density of the culture at 620 nm was monitored using a spectrophotometer. When cultures reached the transition between log and stationary growth (change in optical density of less than 5% in 15 minutes), they were used for transformation with DNA.

Donor DNA was obtained from B. subtilis Strain IA221 (ATCC 39,086) by the following procedure.

Strain IA221 was grown in 100 ml Penassay Broth[R] (Difco). The mixture was grown at 37°C until the optical density of the mixture measured at 660 nanometers was 0.6. The cells were collected by centrifugation and resuspended in 1 ml of a solution containing 0.005 M ethylenediamine tetraacetic acid[R] (EDTA) and 0.05 M NaCl containing 2 mg of lysozyme. When the cells began to lyse, they were frozen by immersing their container in a dry ice-acetone bath. Then 5 ml of a buffer solution containing 0.03 M tris(hydroxymethyl)-aminomethane at pH 8.0 and 0.005 M EDTA[R] was added to the frozen cells and the mixture was frozen and thawed two times. An equal volume of phenol was added and the mixture was shaken for 70 minutes at 4°C. The precipitated protein was removed by centrifugation. DNA was precipitated from the clear upper layer by the addition of 0.12 ml of 3 M sodium acetate (pH 8.0) and 0.64 ml isopropanol per ml of solution. The precipitated DNA was collected by winding on a glass rod and washed with isopropanol. The DNA was redissolved in a solution containing 0.015 M sodium chloride and 0.0015 sodium acetate at pH 7 and stored at 4°C before it was used for transformation.

Competent cells of B. subtilis Strain IA289 (1.0

ml) prepared as described above, were mixed with DNA at a concentration of 10 μg/ml of final mixture and the culture was shaken gently (100 rpm) for 30 minutes at 37°C. The culture was then diluted with 2 ml of Penassay Broth[R] (Difco) and allowed to grow for an additional 90 minutes at 37°C. The cells were collected by centrifugation, washed once with distilled water, resuspended in the original volume of medium and spread on plates containing agar with Spizizen's minimal medium supplemented with methionine (5 μg/ml). Spizizen's minimal medium is a solution of (a) ammonium sulfate 0.2%; (b) potassium phosphate (dibasic) - 1.4%; (c) potassium phosphate (monobasic) - 0.6%; (d) sodium citrate - 0.1%; and (e) magnesium sulfate - 0.02%; pH adjusted to 7.4. Of the two colonies which grew when 0.5 ml of cell suspension was added, one did not produce amylase. It was selected based on its ability to grow in the absence of supplied aromatic amino acids and designated as B1-20. It had the markers: metB5, amyE, and sacA321. A biologically pure culture of this strain is on deposit at the American Type Culture Collection as ATCC 39,706.

Strain B1-20 (metB5, amyE, sacA321) was converted into the asporogenous strain, B1-109, by transformation with DNA from the donor strain, 1S30 (ATCC 39,712) . Transformation was carried out using competent B1-20 cells.

Donor DNA was obtained from B. subtilis Strain 1S30 (ATCC 39,712) by the following procedure. Cells were grown overnight at 37°C in 100 ml of a Tryptic Soy with glucose (TSG) medium[R] (Difco). Then 40 mg of lysozyme was added, and the mixture was incubated for 30 minutes at 60°C. The spheroplasts were separated by centrifugation for 10 minutes at 4500 rpm and suspended in 40 ml of a solution containing 0.15 M NaCl and 0.1 M EDTA at pH 10.2. After 3 ml of 25% sodium dodecyl sulfate solution was added, the mixture was incubated at 70°C for 1 hour. The protein was precipitated twice by additions of a solution containing 0.03 M NaCl and 0.003 M sodium citrate at pH 7 saturated with phenol. To the supernatant was added 5 ml of 3 M sodium acetate solution. A layer of isopropanol was added to the solution. DNA which formed at the interface was wound onto a glass rod. The DNA on the rod was washed twice with cold isopropanol and dissolved in a solution containing 0.03 M NaCl and 0.003 M sodium citrate at pH 7. The resulting solution was dialyzed against three changes of a large volume of a solution containing 0.015 M NaCl and 0.0015 M sodium citrate at pH 7 during a 24-hour period before it was used for transformation.

Cells of B. subtilis Strain BI-20 were grown at 37°C overnight on plates containing Tryptose Blood Agar Base[R] (Difco). Cells from the plates were then suspended in a small amount of growth medium and used to inoculate about 60 ml of additional growth medium in a 500-ml Erlenmeyer flask. Mixture was grown at 33-37° C with shaking. The growth medium contained the following ingredients: 0.5% glucose, 0.6% KH$_2$PO$_4$, 1.4% K$_2$HPO$_4$, 0.1% sodium citrate, 0.2% (NH$_4$)$_2$SO$_4$, 0.01% MgSO4, 0.02% Casamino acids (Difto), 0.1% yeast extract (Difco) and 0.02% L-tryptophan. Growth was monitored by following optical density at 620 nm. When cultures reached the transition between log and stationary growth (change in optical density less than 5% in 15 minutes), they were used for transformation with DNA. The suspension of cells was diluted with 2 volumes of a transformation medium which had the same composition as the growth medium except that it contained an additional 2% of 0.1 M MgCl$_2$ and 1% of 0.05 M CaCl$_2$. Dilution into prewarmed transformation medium at 33-37° C was performed. The mixture was incubated at this temperature for 90 minutes with shaking at 300 rpm. Five minutes prior to the end of the incubation period sterile 20 mM ethylene glycol-bis(beta-aminoethyl ether)-N,N,N',N'-tetraacetic acid was added to give a final concentration of 1 mM.

Transformation was accomplished by mixing 20 µl of DNA isolated from Strain IS30 as described above with 0.5 ml of competent cells of Strain BI-20 described above. The mixture was incubated at 30-37° C for 90 minutes with shaking at 250 rpm. The cultures were plated on agar plates after dilution with sterile water. Cells showing methionine-positive, amylase-negative characteristics were selected by growing cultures on agar plates containing Spizizen's minimal medium supplemented with tryptophan (5 µg/ml) and 1% Lintner starch. Six hundred colonies were then screened for asporogenic characteristics by heat shocking them for 10 minutes at 90° C. One colony, BI-109, was asporogenic. It has the genetic markers: spoIIA12, amyE, and sacA321. A biologically pure culture of this strain is on deposit at the American Type Culture Collection as ATCC 39,701.

Strain BI-109 requires mineral salts containing ammonium, potassium, phosphate and sodium ions for growth. It will utilize various carbon sources including glucose. The strain does not revert to spore formers as shown by the following test. Cells were grown on agar plates containing DSM$^R$ (Difco) medium at 37° C. After 24 hours, the cells were suspended in a tryptic soy medium containing 0.1% glucose and heated at 90° C for 10 minutes. The cells were then titered on plates containing Tryptose Blood Agar Base$^R$ (Difco). Total viable cells were determined as colony-forming units on these plates. Comparisons were made with a number of colony-forming units per milliliter developed when unheated samples were titered. The unheated samples contained 1.27 x 10$^8$ colony-forming units per ml, whereas, the heated cells contained only 5 colony-forming units per ml. This indicates that the strain has a frequency of reversion to spore formers of less than about 10$^{-7}$ when grown under conditions of aeration.

Both strains, BI-20 and BI-109, show a high degree of competence for plasmids in classical transformation reactions. The plasmid vectors are retained in these hosts even when the hosts with the vectors are grown at temperatures as high as 45° C.

The work described herein was all done in conformity with physical and biological containment requirements specified in the NIH guidelines.

## Claims

1. A culture of asporogenous B. subtilis ATCC No. 39,701 suitable for use as a host component in a host-vector system characterized in that it contains no amylase-coding gene, has a frequency of reversion to spore formers of less than about 10$^{-7}$ when grown under conditions of aeration and having the following genetic markers: spoIIA12, amyE, and sacA321.

2. A culture of amylase-negative B. subtilis ATCC No. 39,706 suitable for use as a host component in a host-vector system, and useful as an intermediate for preparation of other amylase-negative host components of host-vector systems characterised in that it contains the genetic markers: metB5, amyE, and sacA321.

3. The process for preparing a strain of B. subtilis having the genetic markers: metB5, amyE, and sacA321, which comprises transforming competent cells of B. subtilis Strain ATCC No. 39,711 with DNA from B. subtilis Strain ATCC No. 39,086 and isolating cells that do not produce alpha-amylase enzyme.

4. The process for preparing a strain of B. subtilis having the genetic markers: spoIIA12, amyE, and sacA321, which comprises transforming competent cells of B. subtilis Strain ATCC No. 39,706 with DNA from B. subtilis Strain ATCC No. 39,712 and isolating cells that grow in the absence of added methionine, do not produce alpha-amylase enzyme, and that do not form spores when heat shocked at 90° C for 10 minutes.

5. The process for using B. subtilis ATCC No. 39,701 as a host in a host-vector system which comprises transforming competent cells of B.

subtilis ATCC No. 39,701 with a plasmid containing desired genetic material and growing the cells containing the plasmid under conditions whereby the plasmid is maintained within the cells.

6. The process for using B. subtilis ATCC No. 39,706 as a host in a host-vector system which comprises transforming competent cells of B. subtilis ATCC No. 39.706 with a plasmid containing desired genetic material and growing the cells containing the plasmid under conditions whereby the plasmid is maintained within the cells.

**Revendications**

1. Culture de B. subtilis asporogène ATCC N° 39 701 convenant pour l'utilisation comme hôte dans un système hôte-vecteur, caractérisée en ce qu'elle ne contient aucun gène codant pour l'amylase, possède une fréquence de réversion vers des organismes sporogènes inférieure à environ $10^{-7}$ lorsqu'elle est cultivée dans des conditions d'aération, et comprend les marqueurs génétiques suivants : spoIIA12, amyE et sacA321.

2. Culture de B. subtilis amylase-négatif ATCC N° 39 706 convenant pour l'utilisation comme hôte dans un système hôte-vecteur et utile comme intermédiaire pour la préparation d'autres hôtes amylase-négatifs de systèmes hôte-vecteur, caractérisée en ce qu'elle contient les marqueurs génétiques : metB5, amyE et sacA321.

3. Procédé de préparation d'une souche de B. subtilis comprenant les marqueurs génétiques : metB5, amyE et sacA321, qui consiste à transformer des cellules compétentes de la souche ATCC N° 39 711 de B. subtilis avec de l'ADN provenant de la souche ATCC N° 39 086 de B. subtilis, et à isoler les cellules qui ne produisent pas l'enzyme alpha-amylase.

4. Procédé de préparation d'une souche de B. subtilis comprenant les marqueurs génétiques : spoIIA12, amyE et sacA321, qui consiste à transformer des cellules compétentes de la souche ATCC N° 39 706 de B. subtilis avec de l'ADN provenant de la souche ATCC N° 39 712 de B. subtilis, et à isoler les cellules qui présentent une croissance en l'absence de méthionine ajoutée, ne produisent pas l'enzyme alpha-amylase et ne forment pas de spores lorsqu'elles sont soumises à un choc thermique à 90° C pendant 10 minutes.

5. Procédé d'utilisation de B. subtilis ATCC N° 39 701 comme hôte dans un système hôte-vecteur, qui consiste à transformer des cellules compétentes de B. subtilis ATCC N° 39 701 avec un plasmide contenant le matériel génétique désiré, et à cultiver les cellules contenant le plasmide dans des conditions permettant le maintien du plasmide à l'intérieur des cellules.

6. Procédé d'utilisation de B. subtilis ATCC N° 39 706 comme hôte dans un système hôte-vecteur, qui consiste à transformer des cellules compétentes de B. subtilis ATCC N° 39 706 avec un plasmide contenant le matériel génétique désiré, et à cultiver les cellules contenant le plasmide dans des conditions permettant le maintien du plasmide à l'intérieur des cellules.

**Ansprüche**

1. Kultur von asporogenem B. subtilis ATCC No. 39701, welche geeignet ist zur Verwendung als Wirt-Komponente in einem Wirt-Vektor-System, dadurch gekennzeichnet, dass sie kein Amylase-kodierendes Gen enthält, eine Reversionsfrequenz zu Sporenbildnern von weniger als etwa $10^{-7}$ aufweist, wenn sie unter Belüftungsbedingungen gezüchtet wird, und die folgenden genetischen Marker besitzt: spoIIA12, amyE und sacA321.

2. Kultur von Amylase-negativem B.subtilis ATCC No. 39706, geeignet zur Verwendung als Wirt-Komponente in einem Wirt-Vektor-System und nützlich als Zwischenprodukt für die Herstellung anderer Amylasenegativer Wirt-Komponenten von Wirt-Vektor-Systemen, dadurch gekennzeichnet, dass sie die genetischen Marker: metB5, amyE und sacA321 enthält.

3. Verfahren zur Herstellung eines Stammes von B.subtilis, welcher die genetischen Marker: metB5, amyE und sacA321 aufweist, dadurch gekennzeichnet, dass kompetente Zellen von B.subtilis, Stamm ATCC No. 39711 mit DNA aus B.subtilis, Stamm ATCC No. 39086 transformiert werden und Zellen, welche kein ß-Amylaseenzym erzeugen, isoliert werden.

4. Verfahren zur Herstellung eines Stammes von B.subtilis, welcher die genetischen Marker: spoIIA12, amyE und sacA321 aufweist, dadurch gekennzeichnet, dass kompetente Zellen von B.subtilis, Stamm ATCC No. 39706 mit DNA aus B.subtilis, Stamm ATCC No. 39712 transformiert werden und Zellen isoliert werden, welche in Abwesenheit von zugesetztem Methionin wachsen, kein ß-Amylaseenzym erzeugen und keine Sporen bilden, wenn sie bei 90°C während 10 Minuten einem Hitzeschock unterworfen werden.

5. Verfahren zur Verwendung von B.subtilis, ATCC No. 39701 als ein Wirt in einem Wirt-Vektor-System, dadurch gekennzeichnet, dass kompetente Zellen von B.subtilis, ATCC No. 39701 mit einem Plasmid, welches das gewünschte genetische Material enthält, transformiert werden und die Zellen, welche das Plasmid enthalten, unter Bedingungen gezüchtet werden, bei welchen das Plasmid innerhalb der Zellen gehalten wird.

6. Verfahren zur Verwendung von B.subtilis, ATCC No. 39706, als ein Wirt in einem Wirt-Vektor-System, dadurch gekennzeichnet, dass kompetente Zellen von B.subtilis, ATCC No. 39706 mit einem Plasmid, welches das gewünschte genetische Material enthält, transformiert werden und die Zellen, welche das Plasmid enthalten, unter Bedingungen gezüchtet werden, bei welchen das Plasmid innerhalb der Zellen gehalten wird.